(19) Europäisches Patentamt
European Patent Office
Office européen des brevets

(11) EP 2 942 399 B1

(12) **EUROPEAN PATENT SPECIFICATION**

(45) Date of publication and mention of the grant of the patent:
**08.03.2017 Bulletin 2017/10**

(51) Int Cl.:
***C12Q 1/68*** *(2006.01)*

(21) Application number: **14167498.6**

(22) Date of filing: **08.05.2014**

(54) **Method for the diagnosis of breast cancer**

Verfahren zur Diagnose von Brustkrebs

Méthode pour le diagnostique du cancer du sein

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.11.2015 Bulletin 2015/46**

(73) Proprietors:
- **Université de Liège**
  **4031 Angleur (BE)**
- **Centre Hospitalier Universitaire de Liège**
  **4000 Liège (BE)**

(72) Inventors:
- **Freres, Pierre**
  **4000 Liege (BE)**
- **Josse, Claire**
  **4000 Liege (BE)**
- **Wenric, Stéphane**
  **4000 Liege (BE)**
- **Jerusalem, Guy**
  **4000 Liege (BE)**
- **Bours, Vincent**
  **4000 Liege (BE)**

(56) References cited:
**EP-A1- 2 341 145 WO-A1-2012/115885**

- **IORIO MARILENA V ET AL: "MicroRNA gene expression deregulation in human breast cancer", CANCER RESEARCH, AMERICAN ASSOCIATION FOR CANCER RESEARCH, US, vol. 65, no. 16, 15 August 2005 (2005-08-15), pages 7065-7070, XP002603432, ISSN: 0008-5472, DOI: 10.1158/0008-5472.CAN-05-1783**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 942 399 B1

## Description

## FIELD OF THE INVENTION

The present invention relates to an in vitro method of diagnosing whether a subject has, or is at risk for developing, breast cancer. The invention also relates to methods of monitoring the progress of breast cancer and of treatment of breast cancer in a subject. The present invention further relates to a kit for use in said methods.

## BACKGROUND OF THE INVENTION

Breast cancer is the most frequently diagnosed cancer worldwide with 1.38 million cases recorded in 2008 and the leading cause of cancer death in women.

Early diagnosis of breast cancer is currently possible by mammography followed by invasive core needle biopsy in case of suspected malignancy. However, overdiagnosis rate of screening mammography is evaluated up to 19%, increasing health care costs, and exposing women to harmful anti-cancer therapies, thereby affecting their quality of life. Moreover, X-ray radiations from mammograms may be one of those factors that can actually trigger breast cancer in high-risk women, e.g. women carrying a mutation in the BRCA1 or BRCA2 genes.

MicroRNAs (miRNAs) are noncoding small RNAs that are synthesized inside the cell, and act as negative regulators of gene expression by binding to the 3'-untranslated region (3'-UTR) within target messenger RNAs (mRNAs).

Several studies have revealed that microRNAs expression was deregulated in breast cancer tissues, as well as in the circulation of breast cancer patients. Circulating microRNAs have the advantage of being protected from degradation by lipoprotein vesicles, called exosomes, and their presence remains highly stable compared to other RNA molecules. Circulating microRNAs are therefore easily accessible and can be used as diagnostic markers in multiple cancers.

In breast cancer, it is well known that circulating microRNAs do not reflect the abundance of microRNAs in the tumor of origin (Pigati et al, PLoS ONE 2010, 5(10): e13515; Cookson et al, Cell Oncol (Dordr) 2012 35(4): 301-8). Hence, the principle that the abundance of microRNAs in biological fluids reflects their abundance in the abnormal cell causing cancer is erroneous. Furthermore blood cells are also major contributors to circulating microRNA in cancer patients (Pritchard et al, Cancer Prev Res (Phila) 2012 5(3): 492-7).

Diagnostic methods measuring circulating microRNAs as biomarkers for minimally invasive breast cancer detection have been disclosed in the art. WO2011/110644 discloses a blood-based diagnostic method of breast cancer based on the measurement of a 4 microRNA signature exhibiting an AUC (Area Under the Curve, a measure of diagnosis accuracy) of 0.932. The accuracy of this method is thus far from being fully effective.

Another limitation of diagnostic methods based on circulating microRNAs measurement is normalization of circulating microRNAs expression level, because a universal housekeeping endogenous microRNA has yet to be identified in the circulation. WO2011/110644 discloses the use of one endogenous miRNA, miR-16, for normalization. However it is well-known that miR-16 is predominantly derived from erythrocytes and has been shown to be prone to artificial elevation by hemolysis, as high as 30 folds (Leidner et al, PLoS ONE 2013, 8(3): e57841), which is a problem for accurate normalization of miRNAs expression levels. The use of blood cells-derived housekeeping microRNAs, such as miR-16, for normalization may be moreover problematic in case of anemia, a condition often occurring in breast cancer patients.

On the other hand, WO2013107459 teaches the use of the mean expression level of the 120 most expressed microRNAs in blood samples for normalization, and Mestdagh et al (Genome Biol 2009, 10:R64) discloses the use of the mean expression value of all commonly expressed microRNAs in a given sample as normalization factor. However, these two approaches are constraining, costly and unsuitable for routine diagnostic testing.

From the above, it is clear that new methods based on circulating microRNAs measurement for breast cancer diagnosis are needed. Ideally they should be minimally invasive, have a higher accuracy to avoid misdiagnosis, and further allow proper normalization of microRNA expression levels for the correct interpretation of results.

## INVENTION DESCRIPTION

We have now invented a minimally invasive and highly accurate in vitro method of diagnosing whether a subject has, or is at risk for developing, breast cancer, or of monitoring the progress of breast cancer based on the measurement of a combination of 8 circulating microRNAs, namely miR-199a-3p, miR-122, miR-29c, miR-33a, miR-324-3p, let-7d, miR-181c and miR-221 in a biological fluid.

The invention hence provides the following embodiments.

In a first embodiment, the invention relates to an in vitro method of diagnosing whether a subject has, or is at risk for developing, breast cancer, comprising the steps:

a. measuring the expression level of the following eight microRNAs selected from the group consisting of: miR-199a-3p, miR-122, miR-29c, miR-33a, miR-324-3p, let-7d, miR-181c or miR-221 in a test biological fluid from said subject;
b. comparing the expression level obtained in step a to a reference value;

wherein an alteration in the expression level of said eight microRNAs is indicative of the subject either having, or being at risk for developing, breast cancer.

This combination of 8 microRNAs surprisingly yielded an AUC of 0.96, when using ten-fold cross-validation on a profiling cohort of 71 breast cancer patients and 14 healthy women. The validity of this classification model has been confirmed on an independent cohort of 30 patients and 6 controls, giving here an AUC of 0.95.

In alternative embodiments of the present invention is provided an in vitro method of diagnosing whether a subject has, or is at risk for developing, breast cancer, comprising the steps:

a. measuring the expression level of microRNAs in a test biological fluid from said subject;
b. comparing the expression level obtained in step a to a reference value;

wherein an alteration in the expression level of said microRNAs is indicative of the subject either having, or being at risk for developing, breast cancer;
wherein said microRNAs are selected from the group comprising:

the following eight microRNAs selected from the group consisting of: miR-122, miR-33a, miR-199a-3p, let-7i*, miR-10a, miR-423-5p, miR-151-5p, miR-221 ;
the following eight microRNAs selected from the group consisting of: miR-122, miR-199a-3p, let-7i*, miR-22*, miR-335, miR-152, miR-151-5p, miR-221 ;
the following seven microRNAs selected from the group consisting of: miR-29c, let-7d, let-7i*, miR-22*, miR-335, miR-152, miR-221 ;
the following eight microRNAs selected from the group consisting of: miR-29c, miR-33a, miR-199a-3p, let-7i*, miR-22*, miR-10a, miR-423-5p, miR-221 ;
or the following nine microRNAs selected from the group consisting of: miR-29c, miR-33a, miR-199a-3p, miR-324-3p, let-7d, let-7i*, miR-335, miR-423-5p, miR-221.

In a preferred alternative embodiment of the method of the present invention, a down-regulation of the expression level of miR-122, miR-33a, miR-199a-3p, let-7i*, miR-10a, miR-423-5p and miR-151-5p and an up-regulation of the expression level of mir-221 is indicative of the subject either having, or being at risk for developing, breast cancer.

In another preferred alternative embodiment of the method of the present invention, a down-regulation of the expression level of miR-122, miR-199a-3p, let-7i*, miR-22*, miR-335 and miR-151-5p and an up-regulation of the expression level of mir-221 and miR-152 is indicative of the subject either having, or being at risk for developing, breast cancer.

In further preferred alternative embodiment of the method of the present invention, a down-regulation of the expression level of miR-29c, let-7d, let-7i*, miR-22* and miR-335 and an up-regulation of the expression level of miR-152 and miR-221 is indicative of the subject either having, or being at risk for developing, breast cancer.

In another further preferred alternative embodiment of the method of the present invention, a down-regulation of the expression level of miR-29c, miR-33a, miR-199a-3p, let-7i*, miR-22*, miR-10a and miR-423-5p and an up-regulation of the expression level of miR-221 is indicative of the subject either having, or being at risk for developing, breast cancer.

In an even another further preferred alternative embodiment of the method of the present invention, a down-regulation of the expression level of miR-29c, miR-33a, miR-199a-3p, let-7d, let-7i*, miR-335 and miR-423-5p and an up-regulation of the expression level of miR-221 and miR-324-3p is indicative of the subject either having, or being at risk for developing, breast cancer.

That is, in each of the preferred embodiments of the methods and kit described in this application the measuring of the expression level of the following eight microRNAs "miR-199a-3p, miR-122, miR-29c, miR-33a, miR-324-3p, let-7d, miR-181c or miR-221" may be replaced by the measuring of the expression level of any one of the following: eight microRNAs: "miR-122, miR-33a, miR-199a-3p, let-7i*, miR-10a, miR-423-5p, miR-151-5p, miR-221"; eight microRNAs: "miR-122, miR-199a-3p, let-7i*, miR-22*, miR-335, miR-152, miR-151-5p, miR-221"; seven microRNAs: "miR-29c, let-7d, let-7i*, miR-22*, miR-335, miR-152, miR-221"; eight microRNAs: "miR-29c, miR-33a, miR-199a-3p, let-7i*, miR-22*, miR-10a, miR-423-5p, miR-221 "; or nine microRNAs: "miR-29c, miR-33a, miR-199a-3p, miR-324-3p, let-7d, let-7i*, miR-335, miR-423-5p, miR-221".

As used herein, the singular forms "a", "an", and "the" include both singular and plural referents unless the context clearly dictates otherwise. By way of example, "a biological fluid" refers to one or more than one biological fluid.

As used herein, the term "subject" can be any mammal. The term "mammal" refers to all mammals, including, but not limited to, humans, dogs, cats, rabbits, ferrets, guinea pigs, mice, rats, hamsters, gerbils, horses, cows and hedgehogs. Preferred mammals are humans. Most preferred mammals are women.

As used herein, the expression "subject at risk for developing breast cancer" refers to subject exhibiting risk factors for breast cancer. Risk factors for breast cancer include, but are not limited to, age, sex, heredity (such as, but not limited to, BRCA1 and BRCA2 mutations), alcohol, fat intake, deregulation in some hormone production, environmental factors, etc. The skilled person is aware of the fact that several different risk factors for breast cancer are disclosed in the art.

The terms microRNA, miRNA or miR are used herein interchangeably, and refer to 19-25 nucleotides mature non-coding RNAs or precursors thereof, or fragments thereof, derived from endogenous genes of living organisms such as animals. Mature microRNAs are processed from longer hairpin-like precursors termed pre-microRNAs (pre-miRs) having a length of approximately 75 nucleotides. MicroRNAs are known from the scientific literature and public databases such as the miRBase database (http://www.mirbase.org). A further property of microRNAs is their presence, in a stable, resistant form, in blood (whole blood, blood serum, blood plasma or blood cells) and in various other biological fluids.

The microRNAs of interest in the present application are incorporated in table 1 below as an example. The skilled person is well aware that microRNAs may be referred to by different names, or synonyms.

Table 1.

| microRNA | Sequence | SEQ ID NO |
|---|---|---|
| let-7b | UGAGGUAGUAGGUUGUGUGGUU | 1 |
| let-7d | AGAGGUAGUAGGUUGCAUAGUU | 2 |
| let-7f | UGAGGUAGUAGAUUGUAUAGUU | 3 |
| let-7g | UGAGGUAGUAGUUUGUACAGUU | 4 |
| let-7i | UGAGGUAGUAGUUUGUGCUGUU | 5 |
| let-7i* | CUGCGCAAGCUACUGCCUUGCU | 6 |
| miR-101 | UACAGUACUGUGAUAACUGAA | 7 |
| miR-103 | AGCAGCAUUGUACAGGGCUAUGA | 8 |
| miR-106a | AAAAGUGCUUACAGUGCAGGUAG | 9 |
| miR-107 | AGCAGCAUUGUACAGGGCUAUCA | 10 |
| miR-10a | UACCCUGUAGAUCCGAAUUUGUG | 11 |
| miR-122 | UGGAGUGUGACAAUGGUGUUUG | 12 |
| miR-125a-5p | UCCCUGAGACCCUUUAACCUGUGA | 13 |
| miR-126 | UCGUACCGUGAGUAAUAAUGCG | 14 |
| miR-126* | CAUUAUUACUUUUGGUACGCG | 15 |
| miR-142-3p | UGUAGUGUUUCCUACUUUAUGGA | 16 |
| miR-142-5p | CAUAAAGUAGAAAGCACUACU | 17 |
| miR-145 | GUCCAGUUUUCCCAGGAAUCCCU | 18 |
| miR-146a | UGAGAACUGAAUUCCAUGGGUU | 19 |
| miR-148b | UCAGUGCAUCACAGAACUUUGU | 20 |
| miR-151-5p | UCGAGGAGCUCACAGUCUAGU | 21 |
| miR-152 | UCAGUGCAUGACAGAACUUGG | 22 |
| miR-15a | UAGCAGCACAUAAUGGUUUGUG | 23 |
| miR-15b | UAGCAGCACAUCAUGGUUUACA | 24 |
| miR-16 | UAGCAGCACGUAAAUAUUGGCG | 25 |
| miR-181a | AACAUUCAACGCUGUCGGUGAGU | 26 |

(continued)

| microRNA | Sequence | SEQ ID NO |
|---|---|---|
| miR-181c | AACAUUCAACCUGUCGGUGAGU | 27 |
| miR-18b | UAAGGUGCAUCUAGUGCAGUUAG | 28 |
| miR-191 | CAACGGAAUCCCAAAAGCAGCUG | 29 |
| miR-199a-3p | ACAGUAGUCUGCACAUUGGUUA | 30 |
| miR-199a-5p | CCCAGUGUUCAGACUACCUGUUC | 31 |
| miR-19a | UGUGCAAAUCUAUGCAAAACUGA | 32 |
| miR-19b | UGUGCAAAUCCAUGCAAAACUGA | 33 |
| miR-20a | UAAAGUGCUUAUAGUGCAGGUAG | 34 |
| miR-21 | UAGCUUAUCAGACUGAUGUUGA | 35 |
| miR-22* | AGUUCUUCAGUGGCAAGCUUUA | 36 |
| miR-221 | AGCUACAUUGUCUGCUGGGUUUC | 37 |
| miR-222 | AGCUACAUCUGGCUACUGGGU | 38 |
| miR-223 | UGUCAGUUUGUCAAAUACCCCA | 39 |
| miR-23a | AUCACAUUGCCAGGGAUUUCC | 40 |
| miR-23b | AUCACAUUGCCAGGGAUUACC | 41 |
| miR-24 | UGGCUCAGUUCAGCAGGAACAG | 42 |
| miR-26a | UUCAAGUAAUCCAGGAUAGGCU | 43 |
| miR-26b | UUCAAGUAAUUCAGGAUAGGU | 44 |
| miR-27a | UUCACAGUGGCUAAGUUCCGC | 45 |
| miR-27b | UUCACAGUGGCUAAGUUCUGC | 46 |
| miR-29c | UAGCACCAUUUGAAAUCGGUUA | 47 |
| miR-30b | UGUAAACAUCCUACACUCAGCU | 48 |
| miR-30c | UGUAAACAUCCUACACUCUCAGC | 49 |
| miR-320a | AAAAGCUGGGUUGAGAGGGCGA | 50 |
| miR-324-3p | ACUGCCCCAGGUGCUGCUGG | 51 |
| miR-335 | UCAAGAGCAAUAACGAAAAAUGU | 52 |
| miR-33a | GUGCAUUGUAGUUGCAUUGCA | 53 |
| miR-423-3p | AGCUCGGUCUGAGGCCCCUCAGU | 54 |
| miR-423-5p | UGAGGGGCAGAGAGCGAGACUUU | 55 |
| miR-425 | AAUGACACGAUCACUCCCGUUGA | 56 |
| miR-451 | AAACCGUUACCAUUACUGAGUU | 57 |
| miR-484 | UCAGGCUCAGUCCCCUCCCGAU | 58 |
| miR-486-5p | UCCUGUACUGAGCUGCCCCGAG | 59 |
| miR-652 | AAUGGCGCCACUAGGGUUGUG | 60 |
| miR-92a | UAUUGCACUUGUCCCGGCCUGU | 61 |
| miR-93 | CAAAGUGCUGUUCGUGCAGGUAG | 62 |

The term "expression level" is any measure for the degree to which the microRNA is produced.

The "expression level" may be determined by measuring the amount of microRNA present in the biological fluid. The expression level of the microRNA can be determined, for example, with an assay for global gene expression in a biological fluid (e.g. using a microarray assay for microRNA expression profiling analysis, or a ready-to-use microRNA qPCR plate), or by specific detection assays, for example, but not limited to, quantitative PCR, quantitative reverse-transcription (real-time) PCR (qRT-PCR), locked nucleic acid (LNA) real-time PCR, or northern blotting. All such assays are well known to those skilled in the art. In particular, the measurement of the expression level of a microRNA in a biological fluid may be carried out with an oligonucleotide probe specific for the detection of said microRNA. Said oligonucleotide probe may bind directly and specifically to the microRNA, or may specifically reverse transcribe said microRNA. Alternatively, said oligonucleotide probe may bind a cDNA obtained from said microRNA. Said oligonucleotide probe may also amplify a cDNA obtained from said microRNA.

The term "biological fluid" refers to a whole blood sample, an urine sample, a milk sample or a saliva sample. It can also refer to a sample derived from whole blood, such as, but not limited to, blood serum, blood plasma or blood cells. The term "biological fluid" does not refer solely to a liquid sample, but can also refer to a liquid sample that has been dried. Hence, for example, biological fluid can refer to dried blood.

Blood samples may be obtained from a subject by various techniques, for example, by using a needle to aspirate a blood sample. Preferably, the sample is obtained in a non-invasive or minimally invasive manner.

The skilled person is well aware as how to isolate blood components, for example blood plasma, blood serum or blood cells.

An alteration in the expression level of a microRNA in a test biological fluid generally occurs if the difference of this expression level to the reference value is statistically significant. For example, an alteration can refer to a down-regulation of the expression level or an up-regulation of the expression level. If the difference is not considered statistically significant, the expression level is considered unchanged. The difference may be considered to be statistically significant if its absolute value exceeds a predetermined threshold value. This threshold value can, for example, be the standard deviation of the expression level found in biological fluids from a population of healthy subjects as indicated in the table below:

| | Expression level in breast cancer patients ($2^{-\Delta Ct}$) | Expression level in healthy subjects ($2^{-\Delta Ct}$) |
|---|---|---|
| miR-122 | 0,15 ± 0,02 | 0,27 ± 0,06 |
| miR-29c | 0,2 ± 0,01 | 0,29 ± 0,02 |
| miR-33a | 0,16 ± 0,02 | 0,33 ± 0,08 |
| miR-199a-3p | 0,31 ± 0,01 | 0,42 ± 0,02 |
| miR-324-3p | 0,088 ± 0,033 | 0,06 ± 0,01 |
| let-7d | 0,085 ± 0,045 | 0,12 ± 0,008 |
| miR-181c | 0,08 ± 0,05 | 0,12 ± 0,009 |
| miR-221 | 1,03 ± 0,46 | 0,73 ± 0,03 |

In a preferred embodiment of the method of the present invention, the reference value is the expression level of the same eight microRNAs in a control biological fluid.

A "control biological fluid" is for example a biological fluid from a subject of the same species not affected by breast cancer, and preferably with no reported history of breast cancer. Alternatively, the reference value may be a previous value for the expression level of a microRNA obtained from a specific subject. This kind of reference value may be used if the method is to be used to monitor the progress of breast cancer, or to monitor the response of a subject to a particular treatment. Preferably, the reference value is the average expression level of the same microRNA found in biological fluids from a population of subjects of the same species not affected by breast cancer. Preferably, said average expression level found in biological fluids from a population of subjects of the same species is determined once and then stored in a database for reference. Preferably, the reference value is measured in biological fluids obtained from one or more subjects of the same species and the same sex and age group as the subject, in which breast cancer is to be diagnosed.

In another preferred embodiment of the method of the present invention, a down-regulation of the expression level of miR-122, miR-29c, miR-33a, miR-199a-3p and let-7d and an up-regulation of the expression level of miR-324-3p, miR-181 c and mir-221 is indicative of the subject either having, or being at risk for developing, breast cancer.

In a further preferred embodiment of the method of the present invention, the expression level of the microRNAs is measured by performing a quantitative reverse-transcription real-time polymerase chain reaction (qRT-PCR).

In a yet another preferred embodiment of the method of the present invention, the expression level in the test or control biological fluid is normalized with a mean expression level of the fifty most expressed microRNAs in said test or control biological fluid.

The term "normalized or "normalization" refers to the comparison of the expression level to one or several control(s) to remove as much variation as possible between biological fluids except for that difference that is a consequence of the breast cancer itself. In the context of the present invention, the expression level is normalized with a mean expression level of the fifty most expressed microRNAs in biological fluids. Many assays are available in the art to determine the most expressed microRNAs in a biological fluid, for example, but non-limited to, using a microarray assay for microRNA expression profiling analysis, or ready-to-use microRNA qPCR plates.

Normalization with a mean expression level of the fifty most expressed microRNAs in the biological fluids has the advantage of avoiding biases occurring when blood cells-derived housekeeping microRNAs, such as miR-16, are used for normalization.

These biases are due to artificial elevation of these blood cells-derived microRNAs caused by hemolysis. Moreover use of blood cells-derived housekeeping microRNAs for normalization may also biases measures in case of anemia, a condition often occurring in breast cancer patients.

Preferably, said most expressed microRNAs in the test and control biological fluid are the same.

More preferably, said most expressed microRNAs are selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

In a yet another preferred embodiment of the method of the present invention, the biological fluid is selected from the group comprising whole blood, blood serum, blood plasma, blood cells, urine, milk or saliva.

Preferred biological fluid is blood plasma.

Blood plasma has the advantage of being a cell-free sample, so its microRNA content is not artificially elevated by hemolysis of blood cells, the number of which may be furthermore modified in breast cancer patients.

In a further preferred embodiment of the method of the present invention, breast cancer is primary or metastatic breast cancer.

A primary breast cancer refers to a breast tumor growing at the anatomical site where tumor progression began, namely the breast, and proceeded to yield a cancerous mass.

Metastatic breast cancer may be a complication of primary breast cancer. It refers to a stage of breast cancer where the disease has spread to distant sites. It is also referred to as, but not limited to, metastases, advanced breast cancer, secondary tumors, secondary or stage 4 breast cancer. Metastatic breast cancer may occur several years after the primary breast cancer, or sometimes may be diagnosed at the same time as the primary breast cancer, or before the primary breast cancer has been diagnosed.

The present invention further relates to an in vitro method of monitoring the progress of breast cancer in a subject comprising the steps of:

a. measuring the expression level of the following eight microRNAs selected from the group consisting of: miR-199a-3p, miR-122, miR-29c, miR-33a, miR-324-3p, let-7d, miR-181c or miR-221 in two or more test biological fluids from said subject, taken at different time intervals.

b. determining the progress of breast cancer by comparing the expression levels of said eight microRNAs in the measured biological fluids over time.

The present invention also relates to a diagnostic kit for use in an in vitro method of diagnosing whether a subject has, or is at risk for developing, breast cancer, or of monitoring the progress of breast cancer in a subject, wherein the kit comprises at least eight oligonucleotide probes specific for the detection of the following eight microRNAs selected from the group consisting of: miR-199a-3p, miR-122, miR-29c, miR-33a, miR-324-3p, let-7d, miR-181c or miR-221.

The term "kit" as used herein refers to any combination of reagents or apparatus that can be used to perform a method of the invention.

The kit may also comprise instructions for use to diagnose whether a subject has, or is at risk for developing, breast cancer, or of monitoring the progress of breast cancer in a subject.

The term "oligonucleotide probe" refers to a short sequence of nucleotides that match a specific region of a microRNA or a cDNA obtained from said microRNA, or fragments thereof, and then used as a molecular probe to detect said microRNA or cDNA sequence.

In the context of the present invention, an oligonucleotide probe "specific for the detection of a microRNA" for example refers to an oligonucleotide probe that bind directly and specifically to a microRNA or a fragment thereof, or specifically reverse transcribe said microRNA. Alternatively, said oligonucleotide probe may bind specifically to a cDNA obtained from said microRNA. Said oligonucleotide probe may also specifically amplify a cDNA obtained from said microRNA.

In a preferred embodiment, the kit further comprises oligonucleotide probes that may be used for normalization purposes. Said oligonucleotides probes are specific for the detection of microRNAs selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

Preferably, oligonucleotide probes are specific for the detection of cDNAs obtained from said microRNAs.

A "cDNA" or "complement DNA" refers to a complementary DNA produced by reverse transcription of an RNA template, such as a microRNA, using a reverse transcriptase enzyme. Examples of reverse transcriptase are reverse transcriptases derived from moloney murine leukemia virus (M-MuLV) reverse transcriptase, avian myeloblastosis virus (AMV) reverse transcriptase, bovine leukemia virus (BLV) reverse transcriptase, Rous sarcoma virus (RSV) reverse transcriptase or human immunodeficiency virus (HIV) reverse transcriptase.

In another preferred embodiment, the kit is adapted for performance of an assay selected from a quantitative reverse-transcription real-time polymerase chain reaction (qRT-PCR), a northern blotting or a micro-array assay.

All such assays are well known to those skilled in the art.

The present invention further relates to the use of the kit described above for diagnosing whether a subject has, or is at risk for developing, breast cancer, or for monitoring the progress of breast cancer in a subject, preferably by performing the methods according to the invention.

The present invention even further relates to a method of treatment of breast cancer in a subject, comprising the steps of:

a. determining whether the subject is in need of receiving breast cancer treatment comprising:

  a1. measuring the expression level of the following eight microRNAs selected from the group consisting of: miR-199a-3p, miR-122, miR-29c, miR-33a, miR-324-3p, let-7d, miR-181c or miR-221 in a test biological fluid from said subject;
  a2. comparing the expression level obtained in step a to a reference value;

wherein an alteration in the expression level of said eight microRNAs is indicative of the subject either having, or being at risk for developing, breast cancer.
b. treating the patient diagnosed in step a as being in need of breast cancer treatment with a treatment selected from the group consisting of: surgery, radiotherapy, chemotherapy, hormonal therapy or biological treatment.

The terms "treatment" or "treating" encompasses both the therapeutic treatment of an already developed breast cancer, as well as prophylactic or preventative measures, wherein the aim is to prevent or lessen the chances of incidence of breast cancer. Beneficial or desired clinical results may include, without limitation, alleviation of one or more symptoms or one or more biological markers, such as, but not limited to, the microRNAs according to the present invention, diminishment of extent of disease, stabilised (*i.e.*, not worsening) state of disease, delay or slowing of disease progression, amelioration or palliation of the disease state, and the like. "Treatment" or "treating" can also mean prolonging survival as compared to expected survival if not receiving treatment.

As used herein, the term "hormonal therapy" refers to the therapeutic use of hormones, for example, but not limited to, the administration of hormones to increase diminished levels in the body, also referred as hormone replacement therapy, or therapy involving the use of drugs or surgical procedures or any other suitable composition or procedure to suppress the production of or inhibit the effects of a hormone. Examples of hormonal therapy include, but are not limited to, the use of selective estrogen receptor modulators (such as tamoxifen), aromatase inhibitors or luteinizing hormone releasing hormone analogues (such as goserelin).

As used herein, the term "biological treatment" refers to the use of living organisms, or substances derived from living organisms, or synthetic versions of such substances to treat breast cancer. Examples of biological treatments include, but are not limited to, antibodies, such as anti-HER2 antibodies, antibody-drug conjugates, antibody fragments, cytokines, therapeutic vaccines, cancer-killing viruses, gene therapy or adoptive T-cell transfer.

In a preferred embodiment of the method of treatment of the invention, the reference value is the expression level of the same eight microRNAs in a control biological fluid.

In another preferred embodiment of the method of treatment of the invention, a down-regulation of the expression level of miR-122, miR-29c, miR-33a, miR-199a-3p and let-7d and an up-regulation of the expression level of miR-324-3p, miR-181c and mir-221 is indicative of the subject either having, or being at risk for developing, breast cancer.

In a further preferred embodiment of the method of treatment of the invention, the expression level in the test or control biological fluid is normalized with a mean expression level of the fifty most expressed microRNAs in said test or control biological fluid.

Preferably, said most expressed microRNAs in the test and control biological fluid are the same.

More preferably, said most expressed microRNAs are selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

The present invention also relates to a kit for use in a method of treatment of breast cancer in a subject, wherein the kit comprises at least eight oligonucleotide probes specific for the detection of the following eight microRNAs selected from the group consisting of: miR-199a-3p, miR-122, miR-29c, miR-33a, miR-324-3p, let-7d, miR-181 c or miR-221.

## BRIEF DESCRIPTION OF THE FIGURES

The present invention is illustrated by the following figures which are to be considered for illustrative purposes only and in no way limit the invention to the embodiments disclosed therein:

**Figure 1:** represents the methodology of the Random forest's algorithm. Step 1: after computing a model on all samples of the profiling cohort (71 patients and 14 controls), with all 188 microRNAs, a ranking of these microRNAs based on 2 importance metrics is obtained (MDA and MDG). 15 different microRNAs were then selected, which were all ranked among the 20 first microRNAs of both rankings. Step 2: based on these 15 microRNAs, 32767 combinations of 2 to 15 microRNAs have been generated. A Random forests based model was built for each of these combinations, and assessed its performance by carrying out a ten-fold cross-validation. This allowed to pick the best performing combination of microRNAs, which are presented in Fig. 2. Step 3: this combination of 8 microRNAs yielded an AUC of 0.96, when using ten-fold cross-validation.

**Figure 2:** represents circulating microRNAs selected for the diagnostic method. (A) 8 microRNAs giving the best performance when combined in a Random forests based model. Performance was assessed by computing the AUC on a ten-fold cross-validation. The rankings of the microRNAs following the two metrics are shown. (B) Expression levels of miR-122 (**, P<0.01), miR-29c (****, P<0.0001), miR-33a (***, P<0.001), miR-199a-3p (***, P<0.001), miR-324-3p (**, P<0.01), let-7d (**, P<0.01), miR-181c (****, P<0.0001) and miR-221 (**, P<0.01) vary significantly in the plasma of 101 breast cancer patients compared to 20 healthy female volunteers. MicroRNAs expression was determined by qRT-PCR. Relative expressions shown are normalized to mean expression value of the 50 most expressed microRNAs. The statistical significance between controls and breast cancer patients were determined by Mann-Whitney U test. Data are expressed as mean ± SEM. (C) ROC Curve obtained by validating the 8-microRNAs signature on the independent cohort (AUC = 0.95; sensitivity = 0.9; specificity = 0.83; threshold = 0.3).

The present invention is further illustrated by the following examples, which do not limit the scope of the invention in any way.

## EXAMPLES

### Materials and Methods

For general methods relating to the invention, reference is made to well-known textbooks, including, e.g. "Current Protocols in Molecular Biology and Short Protocols in Molecular Biology, 3rd Ed." (F. M. Ausubel et al., eds., 1987 & 1995); "MicroRNA Protocols", series: Methods in Molecular Biology, Vol. 936 Ying, Shao-Yao (Ed.) 2nd ed. 2013, XI; "MicroRNA and Cancer, Methods and Protocols", series: Methods in Molecular Biology, Vol. 676 Wu, Wei (Ed.), incorporated by reference herein.

For further elaboration of general techniques useful in the practice of this invention, the practitioner can refer to standard textbooks and reviews in microRNA detection and blood fractionation. Included are "MicroRNA Expression Detection Methods", Wang Zhiguo, Yang Baofeng, 2010, XX; "Circulating MicroRNAs Methods and Protocols", series: Methods in Molecular Biology, Vol. 1024 Ochiya, Takahiro (Ed.) 2013; "Blood separation and plasma fractionation", James R. Harris Wiley-Liss ed., 1991, incorporated by reference herein.

### *Patients, controls, plasma and tissues collection*

Ethical approval was obtained from the institutional board. Patients with new diagnosis of primary or metastatic breast cancer were recruited prospectively at Liege University Hospital from 7/2011 to 7/2013 and have signed a written informed consent. 101 patients have been included in the study. Blood samples were withdrawn in 2 ml EDTA tubes. Plasma was prepared within 1 hour by retaining supernatant after double centrifugation at 4°C (10 min at 815 g and 10 min at 2500 g) and was stored at -80°C.

Plasma were also collected from 23 metastatic breast cancer patients before starting treatments.

Control plasma samples were obtained from 20 healthy female volunteers of similar mean age (51 ± 10 years) without history of cancer.

All patients and tumor characteristics are summarized in Table 2.

Table 2. Patients and tumors characteristics.

| Characteristics | Primary breast cancer patients with NAC (*n* = 36) | Primary breast cancer patients with AC (*n* = 24) | Primary breast cancer patients without CT (*n* = 28) | Metastatic breast cancer patients (*n* = 23) |
|---|---|---|---|---|
| Age (mean ± SD) (y) | 49 ± 14 | 52 ± 10 | 63 ± 13 | 59 ± 11 |
| Estrogen receptor [n (%)] | 21 (58) | 20 (83) | 27 (96) | 19 (83) |
| Progesterone receptor [n (%)] | 17 (47) | 15 (63) | 26 (93) | 10 (43) |
| HER2 [n (%)] | 15 (42) | 7 (29) | 2 (7) | 5 (22) |
| KI-67 (mean ± SD) (%) | 38 ± 21 | 27 ± 19 | 8 ± 7 | 22 ± 22 |
| Molecular subtype [n (%)] | | | | |
| NA | 0 (0) | 0 (0) | 0 (0) | 4 (17) |
| Luminal A | 1 (3) | 3 (12) | 23 (82) | 9 (39) |
| Luminal B | 20 (55) | 17 (71) | 4 (14) | 5 (22) |
| HER2-enriched | 5 (14) | 0 (0) | 1 (4) | 2 (9) |
| ER-negative | 10 (28) | 4 (17) | 0 (0) | 3 (13) |
| Initial T staging [n (%)] | | | | |
| NA | 0 (0) | 0 (0) | 0 (0) | 23 (100) |
| 1 | 1 (3) | 12 (50) | 23 (82) | 0 (0) |
| 2 | 21 (58) | 9 (38) | 5 (18) | 0 (0) |
| 3 | 5 (14) | 3 (12) | 0 (0) | 0 (0) |
| 4 | 9 (25) | 0 (0) | 0 (0) | 0 (0) |
| Size (mean ± SD) (mm) | 48 ± 26 | 27 ± 21 | 16 ± 9 | NA |
| PosttreatmentypT staging [n (%)] | | | | |
| NA | 0 (0) | 24 (100) | 28 (100) | 23 (100) |
| 0 | | 0 (0) | 0 (0) | 0 (0) |
| 1 | | 0 (0) | 0 (0) | 0 (0) |
| 2 | | 0 (0) | 0 (0) | 0 (0) |
| 3 | | 0 (0) | 0 (0) | 0 (0) |
| 4 | | 0 (0) | 0 (0) | 0 (0) |
| Initial N staging [n (%)] | | | | |
| NA | 2 (5) | 0 (0) | 0 (0) | 23 (100) |
| 0 | 11 (31) | 12 (50) | 22 (79) | 0 (0) |
| 1 | 20 (56) | 8 (33) | 6 (21) | 0 (0) |
| 2 | 1 (3) | 3 (13) | 0 (0) | 0 (0) |

(continued)

| Characteristics | Primary breast cancer patients with NAC ($n$ = 36) | Primary breast cancer patients with AC ($n$ = 24) | Primary breast cancer patients without CT ($n$ = 28) | Metastatic breast cancer patients ($n$ = 23) |
|---|---|---|---|---|
| 3 | 2 (5) | 1 (4) | 0 (0) | 0 (0) |
| PosttreatmentypN staging [n (%)] | | | | |
| NA | | 24 (100) | 28 (100) | 23 (100) |
| 0 | | 0 (0) | 0 (0) | 0 (0) |
| 1 | | 0 (0) | 0 (0) | 0 (0) |
| 2 | | 0 (0) | 0 (0) | 0 (0) |
| 3 | | 0 (0) | 0 (0) | 0 (0) |
| Scarff-Bloom-Richardson grading system [n (%)] | | | | |
| NA | 0 (0) | 0 (0) | 0 (0) | 22 (96) |
| 1 | 0 (0) | 0 (0) | 3 (11) | 0 (0) |
| 2 | 15 (42) | 10 (42) | 24 (86) | 1 (4) |
| 3 | 21 (58) | 14 (58) | 1 (3) | 0 (0) |
| Histologic subtype [n (%)] | | | | |
| NA | 0 (0) | 0 (0) | 0 (0) | 0 (0) |
| IDC | 35 (97) | 19 (79) | 19 (68) | 17 (74) |
| ILC | 1 (3) | 4 (17) | 7 (25) | 5 (22) |
| Others | 0 (0) | 1 (4) | 2 (7) | 1 (4) |
| Lymphovascular invasion [n (%)] | 7 (19) | 7 (29) | 3 (11) | NA |
| Trastuzumab [n (%)] | 16 (44) | 7 (29) | 0 (0) | 3 (13) |
| Radiotherapy [n (%)] | 35 (97) | 21 (88) | 22 (79) | 9 (39) |
| Hormonotherapy [n (%)] | | | | |
| SERM | | 9 (38) | 20 (71) | 13 (57) |
| AI | | 11 (46) | 7 (25) | 5 (22) |

***Study design***

The study consists in measuring 188 microRNAs levels in plasma in 36 patients with NAC (= neoadjuvant chemotherapy); 15 with AC (= adjuvant chemotherapy) ; 27 without CT (= chemotherapy) ; 20 healthy women and 23 metastatic breast cancer patients before starting therapies. The microRNAs were chosen on the base of a previous pilot study (data not shown).

***RNA extraction and qRT-PCR of microRNAs***

Circulating microRNAs were purified from 100 µl of plasma with the miRNeasy mini kit (Qiagen, Germany) according to the manufacturer's protocol. The standard protocol was adapted on the basis of Kroh's recommendations (Kroh et al, Methods 2010, 50:298-301), MS2 (Roche, Belgium) was added to the samples as carrier, and cel-miR-39 as spike-in. RNA was eluted in 50 µl RNase-free water at the end of the procedure.

Reverse transcription was performed by using miRCURY LNA™ Universal RT microRNA PCR, polyadenylation and cDNA synthesis kit (Exiqon, Denmark). Quantitative PCR were realized according to the manufacturer's instructions on custom panels of 188 selected microRNAs (Pick-&-Mix microRNA PCR Panels). Controls include reference genes described in the text, inter-plate calibrators (in triplicate) and negative controls.

All PCR reactions were performed on an Applied Biosystems 7900HT Real-Time PCR System (Applied Biosystems). MicroRNAs were considered for analyses with a cycle threshold (Ct) value < 36.

***Data analysis***

Analyses were conducted by the $2^{-\Delta Ct}$ method ($\Delta Ct = Ct_{sample} - Ct_{reference}$ gene) for each sample to obtain normalized expression value.

The reference genes were chosen as following:

*(i) Circulating microRNA on Pick-&-Mix microRNA PCR Panels (Exiqon)*

Data were normalized using ΔCt method as recommended by Mestdagh et al. (2009). The mean Ct of the 50 most expressed microRNAs was used for normalization, as it was the most stable reference gene according to GeNorm software. The analysis with the GeNorm software reveals the best reference gene for accurate normalization in an experimental system by ranking the candidate reference genes according to their expression stability. A gene expression normalization factor (M value) can be calculated based on the geometric mean of a user-defined number of reference genes. The M-value threshold for stability of a gene according to GeNorm is 1.5. As reference gene set, we defined the following combination and compared their M values : all microRNA tested, each taken alone; the global mean of expression values obtained for each microRNA ; the mean of the expression values of the 50 most expressed genes obtained for each microRNA. The last combination has given the best (meaning smaller) M value (table 3). Once the combination of reference genes has been defined, the mean of expression value of those 50 microRNAs is calculated and this mean is used as reference to apply the ΔCT method.

Results of GeNorm analysis are available in table 3.

Table 3: GeNorm analysis of the 50 most expressed microRNAs each taken alone, the mean of the 50 most expressed microRNAs (GM50), the mean of the 120 most expressed microRNAs (GM120) and the mean of all the microRNAs tested (GM188).

| Reference genes | M value |
|---|---|
| GM50 | 0.761 |
| GM120 | 0.767 |
| GM188 | 0.784 |
| miR-93 | 0.854 |
| miR-223 | 0.860 |
| miR-425 | 0.886 |
| miR-103 | 0.893 |
| miR-126 | 0.899 |
| let-7g | 0.901 |
| miR-423-3p | 0.901 |
| miR-142-3p | 0.922 |
| let-7d* | 0.924 |
| miR-222 | 0.924 |
| miR-484 | 0.926 |
| miR-126* | 0.926 |
| miR-26b | 0.945 |
| miR-101 | 0.951 |
| miR-15b | 0.957 |
| miR-146a | 0.962 |
| miR-30b | 0.963 |
| miR-191 | 0.987 |
| miR-15a | 0.990 |
| miR-652 | 1.013 |
| miR-18b | 1.017 |
| miR-21 | 1.034 |
| miR-30c | 1.035 |
| miR-148b | 1.042 |
| let-7f | 1.046 |
| miR-125a-5p | 1.058 |

(continued)

| Reference genes | M value |
|---|---|
| miR-23b | 1.086 |
| miR-26a | 1.091 |
| miR-145 | 1.092 |
| miR-92a | 1.140 |
| miR-221 | 1.141 |
| let-7b | 1.143 |
| miR-27a | 1.152 |
| miR-20a | 1.156 |
| miR-486-5p | 1.161 |
| miR-181a | 1.168 |
| miR-19b | 1.184 |
| miR-16 | 1.191 |
| miR-23a | 1.209 |
| miR-151-5p | 1.221 |
| miR-320a | 1.251 |
| miR-24 | 1.291 |
| miR-199a-5p | 1.325 |
| miR-27b | 1.487 |
| miR-451 | 1.511 |
| miR-150 | 1.637 |
| miR-199a-3p | 1.720 |
| miR-19a | 1.808 |
| miR-106a | 1.844 |
| miR-107 | 1.940 |

Statistical analyses were performed with GraphPad Prism software, version 6.00 (GraphPad Software, USA). Normal distribution of values was evaluated with D'Agastino-Pearson omnibus and Shapiro-Wilk tests. To compare microRNAs levels, Student's t-test (paired or unpaired, two-tailed) and non-parametric tests (Wilcoxon or Mann-Whitney tests, two-sided) were used. Statistical significance was set as P<0.05 (*), P<0.01 (**), P<0.001 (***) or P<0.0001 (****) (Pigati et al, 2010).

In short, the present invention provides a new diagnostic model, based on 8 microRNAs measured in plasma of 71 breast cancer patients and 14 healthy women. The validity of this classification model has been confirmed on an independent cohort of 30 patients and 6 controls.

In particular, the inventors collected the initial expression of the 188 selected microRNAs in breast cancer patients (both primary and metastatic, n = 101) and healthy women (*n* = 20). All individuals were then separated into 2 independent cohorts: an initial diagnostic cohort (profiling cohort, *n* = 85) and a confirmation cohort (validation cohort, *n* = 36). Based on the expression of these 188 microRNAs in the profiling cohort, a diagnostic tool has been designed using a supervised classification algorithm called Random forests. Random forests are an ensemble tree-based supervised learning method that operates by building a large number of decision trees where the chosen features are randomly selected (hence the name, Random forests) (Breiman et al, Machine Learning 2001, 45:5-32). A methodology somewhat similar to Geurts *et al.* has been used (Geurts et al, Bioinformatics 2005, 21:3138-3145) and is explained in Figure 1. Shortly, a model is built and cross-validated using all of the 188 microRNAs, allowing us to rank the microRNAs in terms of their importance in said model (*step 1*). Then, based on this ranking, a selection of microRNAs is made (*step 2*), and a new model is built and cross-validated using only a subset of the 188 microRNAs (*step 3*). This final model is then validated on an independent cohort. This allowed us to pick the best performing combination of microRNAs, which are presented in Figure 2.

An R implementation of Breiman's original Random forests algorithm (Breiman et al, 2001) has been used, provided in the *R* package *randomForest.* The number of trees used in the model (*ntree* parameter of the *randomForest* function of the *R* package) has been determined through a specific approach. For incremental values of *ntree*, a Random forest model is computed, and a ranking for all microRNAs based on both importance metrics, the mean decrease in accuracy (MDA) and the mean decrease in Gini (MDG), is obtained. As soon as these rankings stabilize, we consider the minimal value of *ntree* to be attained. A conservative value of 3000 for *ntree* is chosen for all steps of the construction of Random forests models of our methodology. Through this approach, the observation that the rankings of several microRNAs interchange along iterations can be done.

The same approach has been tried to select an appropriate value for $m_{try}$, but no significant performance change has been seen for values of $m_{try}$ going from 1 to $n$, where $n$ is the total number of microRNAs. Performance was measured here by either the out-of-bag error rate, or the AUC obtained by plotting a ROC curve when doing a cross-validation on the model.

A default value of $m_{try} = \sqrt{(number\ of\ miRNAs)}$ has been chosen.

For the cross-validations performed in *step* 2 and 3 of the methodology (Figure 1), we chose to carry out ten-fold cross validations, as to avoid possible selection bias.

**Results**

***Diagnosis of primary and metastatic breast cancers***

When comparing 188 microRNAs profile of 78 newly diagnosed primary breast cancers to 20 control women, 61 microRNAs are founded significantly deregulated. miR-221 and miR-29c are respectively the most significantly upregulated and downregulated microRNA. A global downregulation of microRNAs is observed in primary breast cancer patients plasma compared to healthy women (0.8 ± 0.3 fold change).

In a second analysis, 188 microRNAs profile from 23 plasma of patients with newly diagnosed metastatic breast cancer were compared to those of 20 healthy women. 68 microRNAs are found significantly deregulated. The most significantly upregulated microRNA is miR-378 and the most significantly downregulated microRNA is miR-199a-3p. As seen in primary breast cancers samples, a global downregulation of microRNAs is observed when compared to healthy subjects (0.9 ± 0.5 fold-change).

Results of statistical analyses for selected microRNAs are summarized in Table 4.

Statistical analyses were also performed to compare both primary and metastatic breast cancer patients to healthy women. 13 microRNAs are significantly modified amoung the 3 groups and these microRNAs are always deregulated in the same way in primary and metastatic breast cancer patients (Table 4).

Table 4. Results of statistical analyses for selected plasma microRNAs. Comparisons between breast cancer patients (both primary and metastatic) and healthy women were performed using Mann-Whitney *U* test. Wilcoxon matched-pairs signed rank test was used to compare microRNAs profiling before and after NAC. NA = not accessed; ns = non significant.

| | Primary breast cancer patients (*n* = 78) *vs*. healthy women (*n* = 20) | | Metastatic breast cancer patients (*n* = 23) *vs*. healthy women (*n* = 20) | | Before *vs*.after NAC (*n* = 25) | |
|---|---|---|---|---|---|---|
| microRNAs | P-value | Fold-change | P-value | Fold-change | P-value | Fold-change |
| let-7d | <0,05 | 0,7 | <0,05 | 0,7 | ns | NA |
| let-7i* | <0,01 | 0,7 | <0,01 | 0,6 | ns | NA |
| miR-106a | ns | NA | <0,05 | 0,3 | <0,05 | 1,4 |
| miR-122 | <0,01 | 0,5 | ns | NA | <0,01 | 1,9 |
| miR-139-5p | ns | NA | <0,001 | 1,4 | <0,001 | 1,5 |
| miR-150 | <0,01 | 0,8 | <0,05 | 0,7 | <0,05 | 0,5 |
| miR-181c | <0,001 | 0,7 | <0,001 | 0,6 | ns | NA |
| miR-199a-3p | <0,05 | 0,8 | <0,0001 | 0,5 | <0,01 | 1,4 |
| miR-221 | <0,001 | 1,5 | ns | NA | ns | NA |
| miR-23a | <0,01 | 1,2 | <0,01 | 1,3 | ns | NA |
| miR-23b | <0,05 | 1,5 | <0,01 | 1,5 | ns | NA |
| miR-29b | <0,05 | 0,8 | <0,0001 | 0,4 | ns | NA |
| miR-29c | <0,001 | 0,7 | <0,0001 | 0,4 | <0,05 | 0,8 |
| miR-324-3p | <0,01 | 1,6 | ns | NA | <0,05 | 0,6 |
| miR-33a | <0,01 | 0,5 | <0,01 | 0,3 | ns | NA |
| miR-34a | <0,05 | 0,8 | ns | NA | <0,0001 | 2,5 |
| miR-378 | ns | NA | <0,0001 | 2,1 | ns | NA |
| miR-409-3p | <0,05 | 1,5 | ns | NA | <0,01 | 0,7 |

(continued)

| | Primary breast cancer patients (*n* = 78) *vs*. healthy women (*n* = 20) | | Metastatic breast cancer patients (*n* = 23) *vs*. healthy women (*n* = 20) | | Before *vs*.after NAC (*n* = 25) | |
|---|---|---|---|---|---|---|
| microRNAs | P-value | Fold-change | P-value | Fold-change | P-value | Fold-change |
| miR-409-5p | <0,05 | 0,8 | <0,01 | 0,7 | ns | NA |
| miR-423-5p | <0,01 | 0,8 | <0,01 | 0,6 | ns | NA |
| miR-451 | ns | NA | <0,01 | 0,5 | <0,01 | 0,5 |
| miR-484 | <0,001 | 1,4 | ns | NA | ns | NA |
| miR-496 | <0,01 | 0,6 | <0,01 | 0,6 | ns | NA |
| miR-503 | ns | NA | ns | NA | <0,01 | 1,6 |

The Random forests method illustrated in Figure 1 allowed the inventors to design a diagnostic method based on the expression of 8 circulating microRNAs, namely miR-199a-3p, miR-122, miR-29c, miR-33a, miR-324-3p, let-7d, miR-181c and miR-221.

This combination of 8 microRNAs yielded an AUC of 0.96, when using ten-fold cross-validation on the profiling cohort. The model has then been validated on an independent cohort, giving here an AUC of 0.95 (Figure 2).

Exact values for sensitivity and specificity can be computed by picking a specific threshold at which the predicted class is chosen. For example, with a threshold of 0.3, a sensitivity of 0.9 and a specificity of 0.83 were obtained. The rankings, importance metrics and plasma expression of the 8 diagnostic microRNAs are shown in Figure 2. One can see that the microRNAs which have the best ranking following the MDA or the MDG are not always the bests in terms of the t-test P-value. This can be explained by the fact that the MDA or the MDG are metrics based on the whole model (ie. they incorporate inter-features relationships). The MDA is a class-specific measure, while the MDG is related to the decrease of the Gini index (it is equal to the sum of all decreases of information in the forest due to a given variable, normalized by the number of trees).

Thus the method of the present invention allows diagnosing primary and metastatic breast cancer with a high accuracy (AUC of at least 0.95; sensitivity = 0.9; specificity = 0.83; threshold = 0.3) compared to methods available in the art. In consequence, the classification model, based on accurate biomarkers, may be very useful to help clinicians to identify patients with high probability of breast cancer without the need of invasive procedures.

**List of abbreviations**

3’-UTR = 3’-untranslated region

AUC = area under the curve

Ct = cycle threshold

DNA = deoxyribonucleic acid

MDA = mean decrease accuracy

MDG = mean decrease Gini

miRNAs = microRNAs

NA = not accessed

NAC = neoadjuvant chemotherapy

Ns = non significant

RNA = ribonucleic acid

SEM = standard error of the mean

## Claims

1. An in vitro method of diagnosing whether a subject has, or is at risk for developing, breast cancer, comprising the steps:

   a. measuring the expression level of the following eight microRNAs: miR-199a-3p, miR-122, miR-29c, miR-33a, miR-324-3p, let-7d, miR-181c or miR-221 in a test biological fluid from said subject;
   b. comparing the expression level obtained in step a) to a reference value;

   wherein an alteration in the expression level of said eight microRNAs is indicative of the subject either having, or being at risk for developing, breast cancer.

2. The in vitro method according to claim 1, wherein said reference value is the expression level of the same eight microRNAs in a control biological fluid.

3. The in vitro method according to any of claims 1 or 2, wherein a down-regulation of the expression level of miR-122, miR-29c, miR-33a, miR-199a-3p and let-7d and an up-regulation of the expression level of miR-324-3p, miR-181c and mir-221 is indicative of the subject either having, or being at risk for developing, breast cancer.

4. The in vitro method according to any of claims 1 to 3, wherein said expression level is measured by performing a quantitative reverse-transcription real-time polymerase chain reaction (qRT-PCR).

5. The in vitro method according to any of claims 1 to 4, wherein said expression level in the test or control biological fluid is normalized with a mean expression level of the fifty most expressed microRNAs in said test or control biological fluid.

6. The in vitro method according to claim 5, wherein said most expressed microRNAs in the test and control biological fluid are the same.

7. The in vitro method according to claims 5 or 6, wherein said most expressed microRNAs are selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

8. The in vitro method according to any of claims 1 to 7, wherein the biological fluid is selected from the group comprising whole blood, blood serum, blood plasma, blood cells, urine, milk or saliva.

9. The in vitro method according to any of claims 1 to 8, wherein breast cancer is primary or metastatic breast cancer.

10. An in vitro method of monitoring the progress of breast cancer in a subject comprising the steps of:

    a. measuring the expression level of the following eight microRNAs: miR-199a-3p, miR-122, miR-29c, miR-33a, miR-324-3p, let-7d, miR-181c or miR-221 in two or more test biological fluids from said subject, taken at different time intervals.
    b. determining the progress of breast cancer by comparing the expression levels of said eight microRNAs in the measured biological fluids over time.

**11.** A kit for use in an in vitro method of diagnosing whether a subject has, or is at risk for developing, breast cancer, or of monitoring the progress of breast cancer in a subject, wherein the kit consists of eight oligonucleotide probes specific for the detection of the following eight microRNAs : miR-199a-3p, miR-122, miR-29c, miR-33a, miR-324-3p, let-7d, miR-181c or miR-221.

**12.** The kit according to claim 11, further comprising oligonucleotide probes specific for the detection of microRNAs selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

**13.** The kit according to any of claims 11 or 12, wherein said oligonucleotide probes are specific for the detection of cDNAs obtained from said microRNAs.

**14.** The kit according to any of claims 11 to 13, adapted for performance of an assay selected from a quantitative reverse-transcription real-time polymerase chain reaction (qRT-PCR), a northern blotting or a micro-array assay.

**15.** Use of a kit for diagnosing whether a subject has, or is at risk for developing, breast cancer, preferably by performing the method according to any of claims 1 to 9, or for monitoring the progress of breast cancer in a subject, preferably by performing the method according to claim 10,
wherein the kit comprises at least eight oligonucleotide probes specific for the detection of the following eight microRNAs : miR-199a-3p, miR-122, miR-29c, miR-33a, miR-324-3p, let-7d, miR-181c or miR-221.

**16.** The use according to claim 15, wherein the kit further comprises oligonucleotide probes specific for the detection of microRNAs selected from the group comprising miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a or miR-19a.

## Patentansprüche

**1.** In-vitro-Verfahren zum Diagnostizieren, ob bei einem Individuum Brustkrebs oder die Gefahr einer Entwicklung davon vorliegt, umfassend die Schritte:

a. Messen des Expressionsniveaus der folgenden acht microRNAs: miR-199a-3p, miR-122, miR-29c, miR-33a, miR-324-3p, let-7d, miR-181c oder miR-221 in einer biologischen Testflüssigkeit aus dem Individuum;
b. Vergleichen des in Schritt a) erhaltenen Expressionsniveaus mit einem Referenzwert;

wobei eine Veränderung des Expressionsniveaus der acht microRNAs anzeigt, dass bei dem Individuum entweder Brustkrebs oder die Gefahr einer Entwicklung davon vorliegt.

**2.** In-vitro-Verfahren nach Anspruch 1, wobei es sich bei dem Referenzwert um das Expressionsniveau der gleichen acht microRNAs in einer biologischen Kontrollflüssigkeit handelt.

**3.** In-vitro-Verfahren nach einem der Ansprüche 1 oder 2, wobei eine Herunterregulierung des Expressionsniveaus von miR-122, miR-29c, miR-33a, miR-199a-3p und let-7d und eine Heraufregulierung des Expressionsniveaus von miR-324-3p, miR-181c und mir-221 anzeigt, dass bei dem Individuum entweder Brustkrebs oder die Gefahr einer Entwicklung davon vorliegt.

**4.** In-vitro-Verfahren nach einem der Ansprüche 1 bis 3, wobei das Expressionsniveau anhand der Durchführung einer quantitativen Reverse-Transkription-Echtzeit-Polymerasekettenreaktion (qRT-PCR) gemessen wird.

**5.** In-vitro-Verfahren nach einem der Ansprüche 1 bis 4, wobei das Expressionsniveau in der biologischen Test- bzw. Kontrollflüssigkeit über ein mittleres Expressionsniveau der fünfzig am stärksten exprimierten microRNAs in der biologischen Test- bzw. Kontrollflüssigkeit normiert wird.

6. In-vitro-Verfahren nach Anspruch 5, wobei die am stärksten exprimierten microRNAs in der biologischen Test- und Kontrollflüssigkeit identisch sind.

7. In-vitro-Verfahren nach Anspruch 5 oder 6, wobei die am stärksten exprimierten microRNAs aus der miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a oder miR-19a umfassenden Gruppe ausgewählt sind.

8. In-vitro-Verfahren nach einem der Ansprüche 1 bis 7, wobei die biologische Flüssigkeit aus der Vollblut, Blutserum, Blutplasma, Blutzellen, Urin, Milch oder Speichel umfassenden Gruppe ausgewählt ist.

9. In-vitro-Verfahren nach einem der Ansprüche 1 bis 8, wobei es sich bei dem Brustkrebs um primären oder metastatischen Brustkrebs handelt.

10. In-vitro-Verfahren zum Überwachen des Fortschreitens einer Brustkrebserkrankung bei einem Individuum, umfassend die Schritte:

a. Messen des Expressionsniveaus der folgenden acht microRNAs: miR-199a-3p, miR-122, miR-29c, miR-33a, miR-324-3p, let-7d, miR-181c oder miR-221 in zwei oder mehr biologischen Testflüssigkeiten aus dem Individuum, die in unterschiedlichen Zeitabständen entnommen wurden;
b. Bestimmen des Fortschreitens einer Brustkrebserkrankung durch Vergleichen der Expressionsniveaus der acht microRNAs in den gemessenen biologischen Flüssigkeiten im zeitlichen Verlauf.

11. Kit zur Verwendung bei einem In-vitro-Verfahren zum Diagnostizieren, ob bei einem Individuum Brustkrebs oder die Gefahr einer Entwicklung davon vorliegt, oder zum Überwachen des Fortschreitens einer Brustkrebserkrankung bei einem Individuum, wobei das Kit aus acht Oligonukleotidsonden besteht, die für den Nachweis der folgenden acht microRNAs spezifisch sind: miR-199a-3p, miR-122, miR-29c, miR-33a, miR-324-3p, let-7d, miR-181c oder miR-221.

12. Kit nach Anspruch 11, ferner umfassend Oligonukleotidsonden, die für den Nachweis von microRNAs spezifisch sind, die aus der miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a oder miR-19a umfassenden Gruppe ausgewählt sind.

13. Kit nach einem der Ansprüche 11 oder 12, wobei die Oligonukleotidsonden für den Nachweis von aus den microRNAs erhaltenen cDNAs spezifisch sind.

14. Kit nach einem der Ansprüche 11 bis 13, adaptiert für die Durchführung eines aus einer quantitativen Reverse-Transkription-Echtzeit-Polymerasekettenreaktion (qRT-PCR), einem Northern-Blot-Verfahren oder einem Mikroarray-Testverfahren ausgewählten Testverfahrens.

15. Verwendung eines Kits zum Diagnostizieren, ob bei einem Individuum Brustkrebs oder die Gefahr einer Entwicklung davon vorliegt, vorzugsweise anhand der Durchführung des Verfahrens nach einem der Ansprüche 1 bis 9, oder zum Überwachen des Fortschreitens einer Brustkrebserkrankung bei einem Individuum, vorzugsweise anhand der Durchführung des Verfahrens nach Anspruch 10,
wobei das Kit wenigstens acht Oligonukleotidsonden umfasst, die für den Nachweis der folgenden acht microRNAs spezifisch sind: miR-199a-3p, miR-122, miR-29c, miR-33a, miR-324-3p, let-7d, miR-181c oder miR-221.

16. Verwendung nach Anspruch 15, wobei das Kit ferner Oligonukleotidsonden umfasst, die für den Nachweis von microRNAs spezifisch sind, die aus der miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a oder miR-19a umfassenden Gruppe ausgewählt sind.

## Revendications

1. Procédé *in vitro* pour diagnostiquer si un sujet a, ou est à risque de développer, un cancer du sein, comprenant les étapes de :

    a. mesure du taux d'expression des huit microARN suivants : miR-199a-3p, miR-122, miR-29c, miR-33a, miR-324-3p, let-7d, miR-181c ou miR-221 dans un fluide biologique d'essai dudit sujet ;
    b. comparaison du taux d'expression obtenu dans l'étape a) à une valeur de référence ;

    dans lequel une modification du taux d'expression desdits huit microARN est une indication que le sujet a, ou est à risque de développer, un cancer du sein.

2. Procédé *in vitro* selon la revendication 1, dans lequel ladite valeur de référence est le taux d'expression des mêmes huit microARN dans un fluide biologique témoin.

3. Procédé *in vitro* selon l'une quelconque des revendications 1 ou 2, dans lequel une régulation à la baisse du taux d'expression de miR-122, miR-29c, miR-33a, miR-199a-3p et let-7d et une régulation à la hausse du taux d'expression de miR-324-3p, miR-181c et mir-221 est une indication que le sujet a, ou est à risque de développer, un cancer du sein.

4. Procédé *in vitro* selon l'une quelconque des revendications 1 à 3, dans lequel ledit taux d'expression est mesuré par conduite d'une réaction de polymérase en chaîne en temps réel par transcription inverse quantitative (qRT-PCR).

5. Procédé *in vitro* selon l'une quelconque des revendications 1 à 4, dans lequel ledit taux d'expression dans le fluide biologique d'essai ou témoin est normalisé avec un taux d'expression moyen des cinquante microARN les plus exprimés dans ledit fluide biologique d'essai ou témoin.

6. Procédé *in vitro* selon la revendication 5, dans lequel lesdits microARN les plus exprimés dans le fluide biologique d'essai et témoin sont les mêmes.

7. Procédé *in vitro* selon les revendications 5 ou 6, dans lequel lesdits microARN les plus exprimés sont choisis dans le groupe comprenant miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a ou miR-19a.

8. Procédé *in vitro* selon l'une quelconque des revendications 1 à 7, dans lequel le fluide biologique est choisi dans le groupe comprenant le sang total, le sérum sanguin, le plasma sanguin, des cellules sanguines, l'urine, le lait ou la salive.

9. Procédé *in vitro* selon l'une quelconque des revendications 1 à 8, dans lequel le cancer du sein est un cancer du sein primitif ou metastatique.

10. Procédé *in vitro* de surveillance de la progression d'un cancer du sein chez un sujet comprenant les étapes de :

    a. mesure du taux d'expression des huit microARN suivants : miR-199a-3p, miR-122, miR-29c, miR-33a, miR-324-3p, let-7d, miR-181c ou miR-221 dans deux fluides biologiques d'essai ou plus dudit sujet, prélevés à des intervalles de temps différents.
    b. détermination de la progression du cancer du sein par comparaison du taux d'expressions desdits huit microARN dans les fluides biologiques mesurés au cours du temps.

11. Ensemble pour utilisation dans un procédé *in vitro* pour diagnostiquer si un sujet a, ou est à risque de développer, un cancer du sein, ou surveiller la progression d'un cancer du sein chez un sujet, l'ensemble étant constitué de huit sondes oligonucléotidiques spécifiques pour la détection des huit microARN suivants : miR-199a-3p, miR-122, miR-29c, miR-33a, miR-324-3p, let-7d, miR-181c ou miR-221.

**12.** Ensemble selon la revendication 11, comprenant en outre des sondes oligonucléotidiques spécifiques pour la détection de microARN choisis dans le groupe comprenant miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a ou miR-19a.

**13.** Ensemble selon l'une quelconque des revendications 11 ou 12, dans lequel lesdites sondes oligonucléotidiques sont spécifiques pour la détection d'ADNc obtenus à partir desdits microARN.

**14.** Ensemble selon l'une quelconque des revendications 11 à 13, adapté pour la conduite d'une réaction de polymérase en chaîne en temps réel par transcription inverse quantitative (qRT-PCR), un transfert Northern ou un dosage sur puce.

**15.** Utilisation d'un ensemble pour diagnostiquer si un sujet a, ou est à risque de développer, un cancer du sein, de préférence par conduite du procédé selon l'une quelconque des revendications 1 à 9, ou pour surveiller la progression d'un cancer du sein chez un sujet, de préférence par conduite du procédé selon la revendication 10,
où l'ensemble comprend au moins huit sondes oligonucléotidiques spécifiques pour la détection des huit microARN suivants : miR-199a-3p, miR-122, miR-29c, miR-33a, miR-324-3p, let-7d, miR-181c ou miR-221.

**16.** Utilisation selon la revendication 15, dans laquelle l'ensemble comprend en outre des sondes oligonucléotidiques spécifiques pour la détection de microARN choisis dans le groupe comprenant miR-484, miR-652, miR-148b, miR-106a, miR-425, let-7g, miR-30b, miR-126, miR-103, miR-146a, miR-93, miR-24, miR-18b, miR-151-5p, miR-423-3p, miR-223, miR-15a, miR-142-3p, miR-26b, miR-15b, miR-191, let-7i, let-7f, miR-21, miR-126*, miR-125a-5p, miR-181a, miR-23a, miR-222, miR-23b, miR-30c, miR-101, miR-29c, miR-320a, miR-26a, miR-145, miR-486-5p, miR-16, miR-199a-5p, miR-107, miR-27a, miR-19b, miR-142-5p, miR-221, let-7b, miR-92a, miR-27b, miR-451, miR-20a ou miR-19a.

Fig. 1

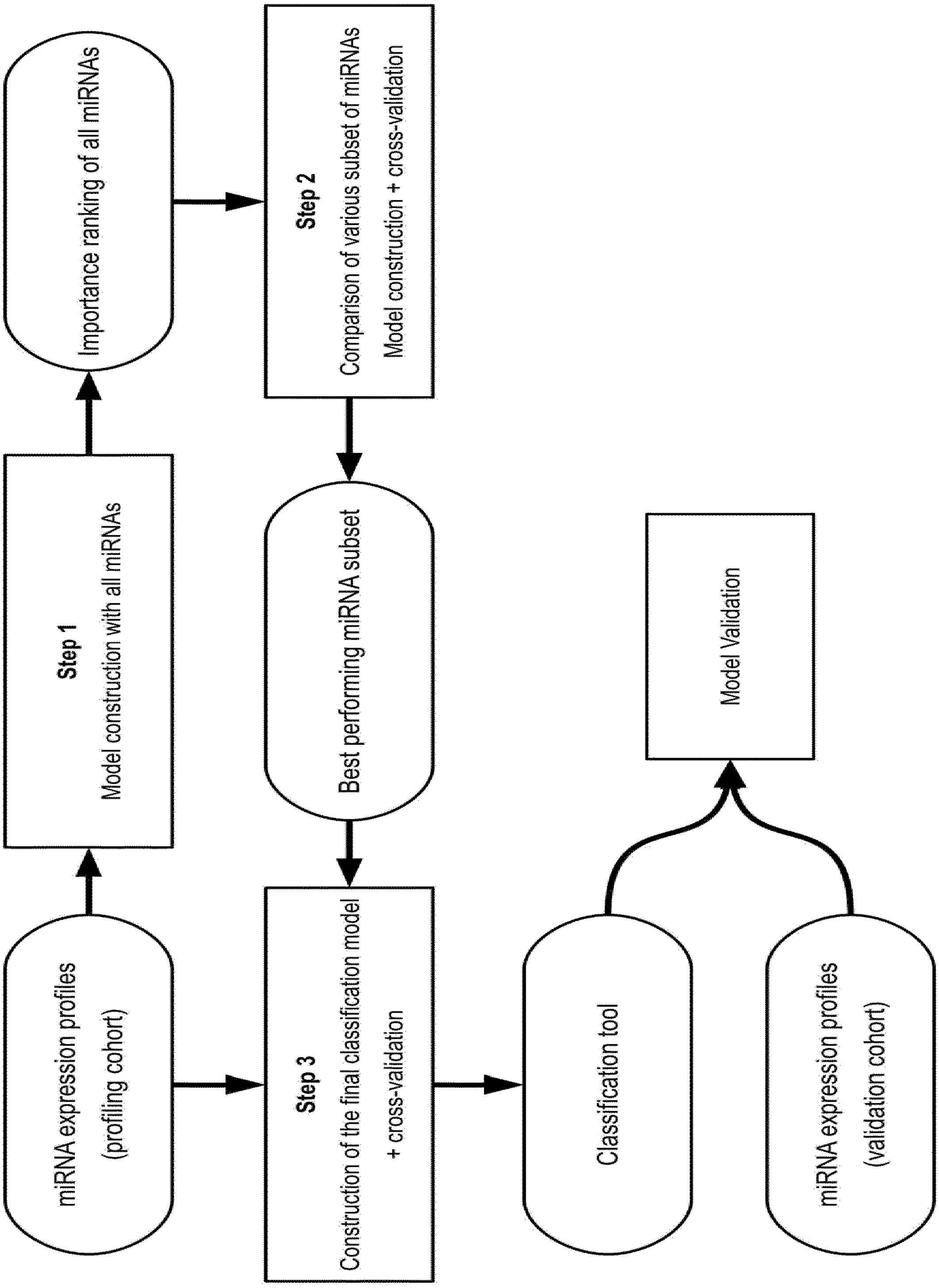
miRNA expression profiles
(profiling cohort)
Step 1
Model construction with all miRNAs
Importance ranking of all miRNAs
Step 2
Comparison of various subset of miRNAs
Model construction + cross-validation
Best performing miRNA subset
Step 3
Construction of the final classification model
+ cross-validation
Classification tool
miRNA expression profiles
(validation cohort)
Model Validation

**Fig. 2 AB**

**A**

| miRNA | Mean Decrease Accuracy (MDA) | MDA ranking | Mean Decrease Gini (MDG) | MDG ranking | P-value (t-test patients *vs.* controls) |
|---|---|---|---|---|---|
| let-7d | 0,001046 | 14 | 0,451241 | 7 | 0,006140 |
| miR-29c | 0,003718 | 2 | 0,596234 | 4 | 0,001070 |
| miR-33a | 0,001783 | 9 | 0,612927 | 2 | 0,043008 |
| miR-122 | 0,006047 | 1 | 0,999151 | 1 | 0,043029 |
| miR-181c | 0,002909 | 3 | 0,467757 | 6 | 0,014575 |
| miR-199a-3p | 0,002900 | 4 | 0,601502 | 3 | $6{,}09 * 10^{-5}$ |
| miR-221 | 0,001630 | 10 | 0,238883 | 19 | 0,000103 |
| miR-324-3p | 0,002338 | 6 | 0,436646 | 8 | 0,035491 |

**B**

**miRNAs expression in plasma**

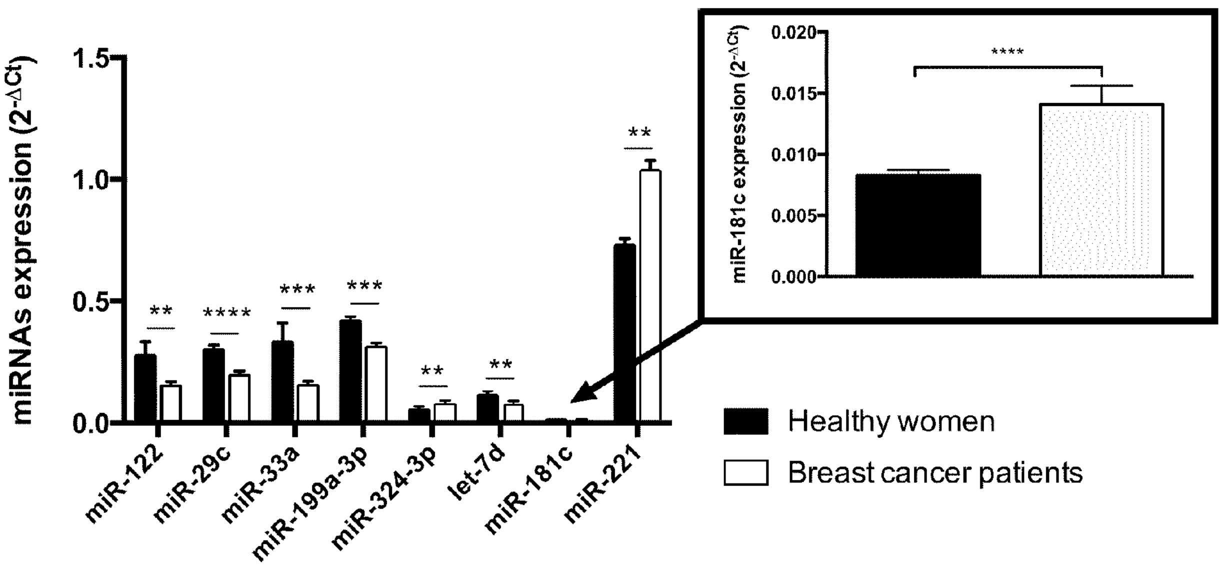

Fig. 2 C

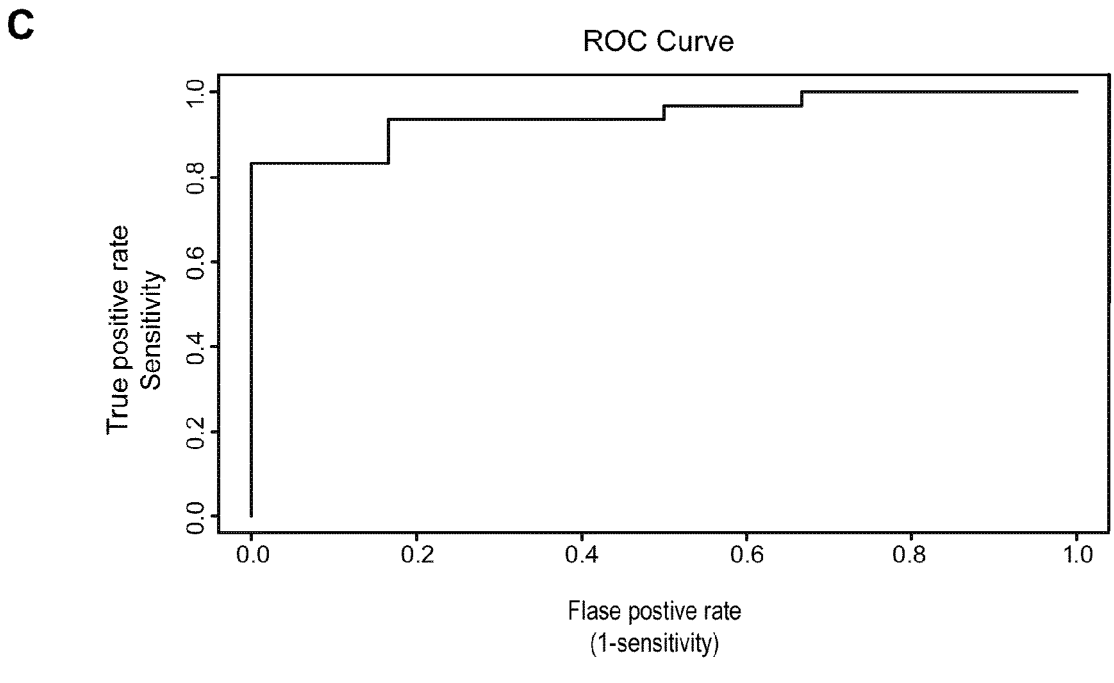
C
ROC Curve
True positive rate
Sensitivity
0.0
0.2
0.4
0.6
0.8
1.0
0.0
0.2
0.4
0.6
0.8
1.0
Flase postive rate
(1-sensitivity)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011110644 A
- WO 2013107459 A


### Non-patent literature cited in the description


- **PIGATI et al.** *PLoS ONE,* 2010, vol. 5 (10), e13515
- **COOKSON et al.** *Cell Oncol (Dordr),* 2012, vol. 35 (4), 301-8
- **PRITCHARD et al.** *Cancer Prev Res (Phila),* 2012, vol. 5 (3), 492-7
- **LEIDNER et al.** *PLoS ONE,* 2013, vol. 8 (3), e57841
- **MESTDAGH et al.** *Genome Biol,* 2009, vol. 10, R64
- Current Protocols in Molecular Biology and Short Protocols in Molecular Biology. 1987
- MicroRNA Protocols. series: Methods in Molecular Biology. 2013, vol. 936, XI
- MicroRNA and Cancer, Methods and Protocols. series: Methods in Molecular Biology. vol. 676
- **WANG ZHIGUO ; YANG BAOFENG.** *MicroRNA Expression Detection Methods,* 2010, XX
- Circulating MicroRNAs Methods and Protocols. series: Methods in Molecular Biology. 2013, vol. 1024
- Blood separation and plasma fractionation. 1991
- **KROH et al.** *Methods,* 2010, vol. 50, 298-301
- **BREIMAN et al.** *Machine Learning,* 2001, vol. 45, 5-32
- **GEURTS et al.** *Bioinformatics,* 2005, vol. 21, 3138-3145

SEQUENCE LISTING

<110> Universite de Liege
Centre Hospitalier Universitaire de Liege

<120> METHOD FOR THE DIAGNOSIS OF BREAST CANCER

<130> 2013-41

<160> 62

<170> BiSSAP 1.2

<210> 1
<211> 22
<212> RNA
<213> Homo sapiens

<220>
<221> source
<222> 1..22
<223> /organism="Homo sapiens"
/mol_type="unassigned RNA"

<400> 1
ugagguagua gguugugugg uu 22

<210> 2
<211> 22
<212> RNA
<213> Homo sapiens

<220>
<221> source
<222> 1..22
<223> /organism="Homo sapiens"
/mol_type="unassigned RNA"

<400> 2
agagguagua gguugcauag uu 22

<210> 3
<211> 22
<212> RNA
<213> Homo sapiens

<220>
<221> source
<222> 1..22
<223> /organism="Homo sapiens"
/mol_type="unassigned RNA"

<400> 3
ugagguagua gauuguauag uu 22

<210> 4
<211> 22
<212> RNA
<213> Homo sapiens

<220>
<221> source
<222> 1..22
<223> /organism="Homo sapiens"
/mol_type="unassigned RNA"

<400> 4
ugagguagua guuuguacag uu 22

<210> 5
<211> 22
<212> RNA
<213> Homo sapiens

<220>
<221> source
<222> 1..22
<223> /organism="Homo sapiens"
/mol_type="unassigned RNA"

<400> 5
ugagguagua guuugugcug uu 22

<210> 6
<211> 22
<212> RNA
<213> Homo sapiens

<220>
<221> source
<222> 1..22
<223> /organism="Homo sapiens"
/mol_type="unassigned RNA"

<400> 6
cugcgcaagc uacugccuug cu 22

<210> 7
<211> 21
<212> RNA
<213> Homo sapiens

<220>
<221> source
<222> 1..21
<223> /organism="Homo sapiens"
/mol_type="unassigned RNA"

<400> 7
uacaguacug ugauaacuga a 21

<210> 8
<211> 23

<212> RNA
<213> Homo sapiens

<220>
<221> source
<222> 1..23
<223> /organism="Homo sapiens"
/mol_type="unassigned RNA"

<400> 8
agcagcauug uacagggcua uga 23

<210> 9
<211> 23
<212> RNA
<213> Homo sapiens

<220>
<221> source
<222> 1..23
<223> /organism="Homo sapiens"
/mol_type="unassigned RNA"

<400> 9
aaaagugcuu acagugcagg uag 23

<210> 10
<211> 23
<212> RNA
<213> Homo sapiens

<220>
<221> source
<222> 1..23
<223> /organism="Homo sapiens"
/mol_type="unassigned RNA"

<400> 10
agcagcauug uacagggcua uca 23

<210> 11
<211> 23
<212> RNA
<213> Homo sapiens

<220>
<221> source
<222> 1..23
<223> /organism="Homo sapiens"
/mol_type="unassigned RNA"

<400> 11
uacccuguag auccgaauuu gug 23

<210> 12
<211> 22
<212> RNA
<213> Homo sapiens

<220>
<221> source
<222> 1..22
<223> /organism="Homo sapiens"
/mol_type="unassigned RNA"

<400> 12
uggaguguga caaugguguu ug 22

<210> 13
<211> 24
<212> RNA
<213> Homo sapiens

<220>
<221> source
<222> 1..24
<223> /organism="Homo sapiens"
/mol_type="unassigned RNA"

<400> 13
ucccugagac ccuuuaaccu guga 24

<210> 14
<211> 22
<212> RNA
<213> Homo sapiens

<220>
<221> source
<222> 1..22
<223> /organism="Homo sapiens"
/mol_type="unassigned RNA"

<400> 14
ucguaccgug aguaauaaug cg 22

<210> 15
<211> 21
<212> RNA
<213> Homo sapiens

<220>
<221> source
<222> 1..21
<223> /organism="Homo sapiens"
/mol_type="unassigned RNA"

<400> 15
cauuauuacu uuugguacgc g 21

<210> 16
<211> 23
<212> RNA
<213> Homo sapiens

<220>
<221> source

<222> 1..23
<223> /organism="Homo sapiens"
/mol_type="unassigned RNA"

<400> 16
uguaguguuu ccuacuuuau gga 23

<210> 17
<211> 21
<212> RNA
<213> Homo sapiens

<220>
<221> source
<222> 1..21
<223> /organism="Homo sapiens"
/mol_type="unassigned RNA"

<400> 17
cauaaaguag aaagcacuac u 21

<210> 18
<211> 23
<212> RNA
<213> Homo sapiens

<220>
<221> source
<222> 1..23
<223> /organism="Homo sapiens"
/mol_type="unassigned RNA"

<400> 18
guccaguuuu cccaggaauc ccu 23

<210> 19
<211> 22
<212> RNA
<213> Homo sapiens

<220>
<221> source
<222> 1..22
<223> /organism="Homo sapiens"
/mol_type="unassigned RNA"

<400> 19
ugagaacuga auuccauggg uu 22

<210> 20
<211> 22
<212> RNA
<213> Homo sapiens

<220>
<221> source
<222> 1..22
<223> /organism="Homo sapiens"

/mol_type="unassigned RNA"

<400> 20
ucagugcauc acagaacuuu gu 22

<210> 21
<211> 21
<212> RNA
<213> Homo sapiens

<220>
<221> source
<222> 1..21
<223> /organism="Homo sapiens"
/mol_type="unassigned RNA"

<400> 21
ucgaggagcu cacagucuag u 21

<210> 22
<211> 21
<212> RNA
<213> Homo sapiens

<220>
<221> source
<222> 1..21
<223> /organism="Homo sapiens"
/mol_type="unassigned RNA"

<400> 22
ucagugcaug acagaacuug g 21

<210> 23
<211> 22
<212> RNA
<213> Homo sapiens

<220>
<221> source
<222> 1..22
<223> /organism="Homo sapiens"
/mol_type="unassigned RNA"

<400> 23
uagcagcaca uaaugguuug ug 22

<210> 24
<211> 22
<212> RNA
<213> Homo sapiens

<220>
<221> source
<222> 1..22
<223> /organism="Homo sapiens"
/mol_type="unassigned RNA"

<400> 24
uagcagcaca ucaugguuua ca 22

<210> 25
<211> 22
<212> RNA
<213> Homo sapiens

<220>
<221> source
<222> 1..22
<223> /organism="Homo sapiens"
/mol_type="unassigned RNA"

<400> 25
uagcagcacg uaaauauugg cg 22

<210> 26
<211> 23
<212> RNA
<213> Homo sapiens

<220>
<221> source
<222> 1..23
<223> /organism="Homo sapiens"
/mol_type="unassigned RNA"

<400> 26
aacauucaac gcugucggug agu 23

<210> 27
<211> 22
<212> RNA
<213> Homo sapiens

<220>
<221> source
<222> 1..22
<223> /organism="Homo sapiens"
/mol_type="unassigned RNA"

<400> 27
aacauucaac cugucgguga gu 22

<210> 28
<211> 23
<212> RNA
<213> Homo sapiens

<220>
<221> source
<222> 1..23
<223> /organism="Homo sapiens"
/mol_type="unassigned RNA"

<400> 28
uaaggugcau cuagugcagu uag 23

<210> 29
<211> 23
<212> RNA
<213> Homo sapiens

<220>
<221> source
<222> 1..23
<223> /organism="Homo sapiens"
/mol_type="unassigned RNA"

<400> 29
caacggaauc ccaaaagcag cug 23

<210> 30
<211> 22
<212> RNA
<213> Homo sapiens

<220>
<221> source
<222> 1..22
<223> /organism="Homo sapiens"
/mol_type="unassigned RNA"

<400> 30
acaguagucu gcacauuggu ua 22

<210> 31
<211> 23
<212> RNA
<213> Homo sapiens

<220>
<221> source
<222> 1..23
<223> /organism="Homo sapiens"
/mol_type="unassigned RNA"

<400> 31
cccaguguuc agacuaccug uuc 23

<210> 32
<211> 23
<212> RNA
<213> Homo sapiens

<220>
<221> source
<222> 1..23
<223> /organism="Homo sapiens"
/mol_type="unassigned RNA"

<400> 32
ugugcaaauc uaugcaaaac uga 23

<210> 33
<211> 23

<212> RNA
<213> Homo sapiens

<220>
<221> source
<222> 1..23
<223> /organism="Homo sapiens"
/mol_type="unassigned RNA"

<400> 33
ugugcaaauc caugcaaaac uga 23

<210> 34
<211> 23
<212> RNA
<213> Homo sapiens

<220>
<221> source
<222> 1..23
<223> /organism="Homo sapiens"
/mol_type="unassigned RNA"

<400> 34
uaaagugcuu auagugcagg uag 23

<210> 35
<211> 22
<212> RNA
<213> Homo sapiens

<220>
<221> source
<222> 1..22
<223> /organism="Homo sapiens"
/mol_type="unassigned RNA"

<400> 35
uagcuuauca gacugauguu ga 22

<210> 36
<211> 22
<212> RNA
<213> Homo sapiens

<220>
<221> source
<222> 1..22
<223> /organism="Homo sapiens"
/mol_type="unassigned RNA"

<400> 36
aguucuucag uggcaagcuu ua 22

<210> 37
<211> 23
<212> RNA
<213> Homo sapiens

<220>
<221> source
<222> 1..23
<223> /organism="Homo sapiens"
/mol_type="unassigned RNA"

<400> 37
agcuacauug ucugcugggu uuc 23

<210> 38
<211> 21
<212> RNA
<213> Homo sapiens

<220>
<221> source
<222> 1..21
<223> /organism="Homo sapiens"
/mol_type="unassigned RNA"

<400> 38
agcuacaucu ggcuacuggg u 21

<210> 39
<211> 22
<212> RNA
<213> Homo sapiens

<220>
<221> source
<222> 1..22
<223> /organism="Homo sapiens"
/mol_type="unassigned RNA"

<400> 39
ugucaguuug ucaaauaccc ca 22

<210> 40
<211> 21
<212> RNA
<213> Homo sapiens

<220>
<221> source
<222> 1..21
<223> /organism="Homo sapiens"
/mol_type="unassigned RNA"

<400> 40
aucacauugc cagggauuuc c 21

<210> 41
<211> 21
<212> RNA
<213> Homo sapiens

<220>
<221> source

<222> 1..21
<223> /organism="Homo sapiens"
/mol_type="unassigned RNA"

<400> 41
aucacauugc cagggauuac c 21

<210> 42
<211> 22
<212> RNA
<213> Homo sapiens

<220>
<221> source
<222> 1..22
<223> /organism="Homo sapiens"
/mol_type="unassigned RNA"

<400> 42
uggcucaguu cagcaggaac ag 22

<210> 43
<211> 22
<212> RNA
<213> Homo sapiens

<220>
<221> source
<222> 1..22
<223> /organism="Homo sapiens"
/mol_type="unassigned RNA"

<400> 43
uucaaguaau ccaggauagg cu 22

<210> 44
<211> 21
<212> RNA
<213> Homo sapiens

<220>
<221> source
<222> 1..21
<223> /organism="Homo sapiens"
/mol_type="unassigned RNA"

<400> 44
uucaaguaau ucaggauagg u 21

<210> 45
<211> 21
<212> RNA
<213> Homo sapiens

<220>
<221> source
<222> 1..21
<223> /organism="Homo sapiens"

/mol_type="unassigned RNA"

<400> 45
uucacagugg cuaaguuccg c 21

<210> 46
<211> 21
<212> RNA
<213> Homo sapiens

<220>
<221> source
<222> 1..21
<223> /organism="Homo sapiens"
/mol_type="unassigned RNA"

<400> 46
uucacagugg cuaaguucug c 21

<210> 47
<211> 22
<212> RNA
<213> Homo sapiens

<220>
<221> source
<222> 1..22
<223> /organism="Homo sapiens"
/mol_type="unassigned RNA"

<400> 47
uagcaccauu ugaaaucggu ua 22

<210> 48
<211> 22
<212> RNA
<213> Homo sapiens

<220>
<221> source
<222> 1..22
<223> /organism="Homo sapiens"
/mol_type="unassigned RNA"

<400> 48
uguaaacauc cuacacucag cu 22

<210> 49
<211> 23
<212> RNA
<213> Homo sapiens

<220>
<221> source
<222> 1..23
<223> /organism="Homo sapiens"
/mol_type="unassigned RNA"

<400> 49
uguaaacauc cuacacucuc agc 23

<210> 50
<211> 22
<212> RNA
<213> Homo sapiens

<220>
<221> source
<222> 1..22
<223> /organism="Homo sapiens"
/mol_type="unassigned RNA"

<400> 50
aaaagcuggg uugagagggc ga 22

<210> 51
<211> 20
<212> RNA
<213> Homo sapiens

<220>
<221> source
<222> 1..20
<223> /organism="Homo sapiens"
/mol_type="unassigned RNA"

<400> 51
acugccccag gugcugcugg 20

<210> 52
<211> 23
<212> RNA
<213> Homo sapiens

<220>
<221> source
<222> 1..23
<223> /organism="Homo sapiens"
/mol_type="unassigned RNA"

<400> 52
ucaagagcaa uaacgaaaaa ugu 23

<210> 53
<211> 21
<212> RNA
<213> Homo sapiens

<220>
<221> source
<222> 1..21
<223> /organism="Homo sapiens" /mol_type="unassigned RNA"

<400> 53
gugcauugua guugcauugc a 21

<210> 54
<211> 23
<212> RNA
<213> Homo sapiens

<220>
<221> source
<222> 1..23
<223> /organism="Homo sapiens"
/mol_type="unassigned RNA"

<400> 54
agcucggucu gaggccccuc agu 23

<210> 55
<211> 23
<212> RNA
<213> Homo sapiens

<220>
<221> source
<222> 1..23
<223> /organism="Homo sapiens"
/mol_type="unassigned RNA"

<400> 55
ugaggggcag agagcgagac uuu 23

<210> 56
<211> 23
<212> RNA
<213> Homo sapiens

<220>
<221> source
<222> 1..23
<223> /organism="Homo sapiens"
/mol_type="unassigned RNA"

<400> 56
aaugacacga ucacucccgu uga 23

<210> 57
<211> 22
<212> RNA
<213> Homo sapiens

<220>
<221> source
<222> 1..22
<223> /organism="Homo sapiens"
/mol_type="unassigned RNA"

<400> 57
aaaccguuac cauuacugag uu 22

<210> 58
<211> 22

<212> RNA
<213> Homo sapiens

<220>
<221> source
<222> 1..22
<223> /organism="Homo sapiens"
/mol_type="unassigned RNA"

<400> 58
ucaggcucag uccccucccg au 22

<210> 59
<211> 22
<212> RNA
<213> Homo sapiens

<220>
<221> source
<222> 1..22
<223> /organism="Homo sapiens"
/mol_type="unassigned RNA"

<400> 59
uccuguacug agcugccccg ag 22

<210> 60
<211> 21
<212> RNA
<213> Homo sapiens

<220>
<221> source
<222> 1..21
<223> /organism="Homo sapiens"
/mol_type="unassigned RNA"

<400> 60
aauggcgcca cuaggguugu g 21

<210> 61
<211> 22
<212> RNA
<213> Homo sapiens

<220>
<221> source
<222> 1..22
<223> /organism="Homo sapiens"
/mol_type="unassigned RNA"

<400> 61
uauugcacuu gucccggccu gu 22

<210> 62
<211> 23
<212> RNA
<213> Homo sapiens

<220>
<221> source
<222> 1..23
<223> /organism="Homo sapiens"
/mol_type="unassigned RNA"

<400> 62
caaagugcug uucgugcagg uag 23